# EUROPÄISCHE PATENTSCHRIFT

(11) **EP 0 816 489 B1**
(45) Veröffentlichungstag und Bekanntmachung des Hinweises auf die Patenterteilung: **14.06.2006**
(21) Anmeldenummer: 97102967.3
(22) Anmeldetag: 24.02.1997
(51) Int. Cl.: C12N 9/10, C12N 9/12, C12N 1/10

(54) **Nukleotidzuckersynthetisierende Enzyme aus nichtparasitären Protisten**
Nucleotidesugar synthesizing enzyme from non-parasitic protists
Enzyme synthetisant le sucre lié aux nucléotides obtenu à partir de protistes non parasitaires

(30) Priorität: 05.03.1996 DE 19608268
(43) Veröffentlichungstag der Anmeldung: 07.01.1998
(73) Patentinhaber: Nutrinova Nutrition Specialties & Food Ingredients GmbH, 65926 Frankfurt am Main (DE)
(72) Erfinder: Kiy, Thomas, Dr., 65929 Frankfurt (DE); Elling, Lothar, Dr., 52066 Aachen (DE); Kula, Maria, Regina, Prof. Dr., 52382 Niederzier-Hambach (DE)
(74) Vertreter: Luderschmidt, Schüler & Partner GbR

(56) Entgegenhaltungen:
- JOURNAL OF BIOLOGICAL CHEMISTRY, Bd. 240, Nr. 24, 1974, Seiten 7832-7840, XP002040823 V.L. RUDICK AND R.A. WEISMAN: "UDP-Glucose Pyrophosphorylase of A. castellanii"
- JOURNAL OF BACTERIOLOGY, Bd. 120, Nr. 3, 1974, Seiten 1151-1157, XP002040824 G.D. KUEHN: "UDP-Glocose pyrophosphorylase activity and differentiation in the acellular slime mold Physarum polycephalum"
- JOURNAL OF BIOCHEMISTRY, Bd. 83, 1978, Seiten 693-698, XP002040825 D.C. DEMEGLIO AND T.B. FRIEDMAN: "Galactose metabolism in D. discoideum"

## Beschreibung

Nukleotidzuckersythetisierende Enzyme aus einen nichtparasitären Protisten

Die vorliegende Erfindung betrifft nukleotidzuckersynthetisierende Enzyme, d.h. Enzyme mit Nukleotidyltransferase- bzw. Pyrophosphorylaseaktivität, aus nichtparasitären Protisten ausgewählt aus der Klassen Ciliata oder Phytomastigiphorea, welcher entweder Tetrahymena, Colpidium, Potomacus oder Euglena ist, ein Verfahren zu deren Herstellung sowie deren Verwendung zur Herstellung von Nukleotidzuckern.

Oligo- und Polysaccharide spielen in der Natur eine wichtige Rolle. Als Komponenten von Glykaproteinen und Glycolipiden sind sie von essentieller Bedeutung bei zellulären Kommunikationsprozessen und als Bindungstellen für z. B. Antikörper, Lectine und Hormone. Sie bestimmen auch maßgeblich die physikatischen Eigenschaften von Proteinen wie Löslichkeit. Stabilität und 3-D-Struktur. Sie dienen darüber hinaus als Rezeptoren für Viren-, Bakterien- und Protisten-vermittelte Krankheitsbilder,

Mit der Erkennung der vielseitigen Funktionen der Kohlenhydratreste in Glykokonjugaten, wuchs auch das Interesse an der Synthese solcher Verbindungen. Aufgrund ihrer hohen Komplexität und den speziellen Anforderungen an die Chemoselektivität, Regioselektivität und Stereoselektivität ist aber bereits die Synthese niederer Oligosaccharide auf chemischem Wege enorm schwierig. Mit steigender Komplexität der Saccharide nimmt auch die Schwierigkeit, sie mittels einer chemischen Synthese nachzubilden, zu. Aus diesem Grunde liegt es nahe den biologischen Synthesewegen nachzueifern und bei der Synthese komplexer Saccharide auf Enzyme, die meist substrat-, regio- und stereospezifische Reaktionen katalysieren, zurückzugreifen.

Die Oligosaccharidstrukturen von Glycokonjugaten werden in vivo durch Leloir-Glycosyltransforasen synthetisiert, die aktivierte Zucker (Nukleitidzucker) als Donorsubstrate für den regio- und stereoselektiven Transfer benötigen. Die Nukleotidzucker werden von Nukleotidyltransferasen bzw. Pyrophosphorylasen (z.B. EC 2.7.7.9 bis 2.7.7.13, 2.7.7.23, 2.7.7.29, 2.7.7.30) unter Verwendung von Nukleosidtriphosphaten gebildet. Dabei wird der Transfer der Nukleotidylgruppe eines Nukleosidtriphosphats (NTP) auf ein Zuckerphosphat (Zucker(-1-P)) unter Freisetzung von Pyrophosphat (PPi) katalysiert.

Die Verwendung von Oligosaccharidstrukturen als potentielle Therapeutika (Inhibitoren, Antiadhäsiva, Entzündungshemmer, immunogene Stimulatoren etc.) oder Diagnostika (Determinanten, Lektin-Liganden) setzt deren uneingeschränkten synthetischen Zugang voraus.

Die Synthese der Oligosaccharidstrukturen kann grundsätzlich über zwei Ansätze erfolgen; über eine chemische oder eine enzymatische Synthese. Die chemische Synthese ist mit vielen Nachteilen behaftet, da komplexe Strategien unter Verwendung von Schutzgruppentechniken in der Regel zeitaufwendig sind und zu nur geringen Ausbeuten führen. Dies gilt gleichermaßen für die chemische Darstellung der Nukleotidzucker. Aufgrund der aufwendigen Synthese und den häufig schlechten Ausbeuten sind die Kosten aber sehr hoch. So kosten 10 mg chemisch synthetisierte GDP-β-L-Fucose ca. 1000,- DM.

Für die enzymatische Synthese von Oligosaccharidstrukturen stehen bereits eine Reihe von Glycosyltransferasen zur Verfügung. Mehr als hundert Glycosyltransferasen wurden mittlerweile aus Eukarvonten isoliert (Glycobiology, 1995, 5, 463). Eine entscheidende Voraussetzung für die großtechnische Synthese ist der enzymatische Zugang zu den teuren Nukleotidzuckern. Hier bestehen zur Zeit ein hoher Bedarf und ein großes wirtschaftliches Interesse. So konnte z.B. die enzymatische Synthese von GDP-β-L-Fucose mangels eines geeigneten Enzymsystems im technischen Maßstab nicht realisiert werden.

Daher stellte sich die Aufgabe, neue. "mikrobielle" Quellen, die sich in einem großen Maßstab fermentieren lassen, für die Gewinnung von nukleotidzuckersynthetisierenden Enzymen zu finden. Überraschenderweise konnte dabei eine bislang vollkommen außer acht gelassene Organismengruppe - die freilebenden, nicht parasitären Protisten - als neue vielversprechende Produzenten der gesuchten Enzyme identifiziert werden, nämlich Tetrahymena, Colpidium, Potomacus oder Euglena.

Bisher waren lediglich einige Pyrophosphorylasen aus parasitären bzw. pathogenen oder fakultativ-pathogenen Protisten bekannt, wie etwa UDP-N-Acetylglucosamin-Pyrophosphorylase aus Giardia (Mol. Blochem. Parasitol., 1992, 56, 301), UTP-Hexose-1-Phosphat Uridyltransferase (E.C. 2.7.7.10) und UDP-Glucose-Pyrophosphorylase aus Entamoeba histolytica (Mol. Biochem. Parasitol., 1983, 7, 173 und Exp. Parasitol., 1977, 43, 115), UDP-Glucose-Pyrophosphorylase aus Hartmanella culbertsoni (J. Protozool., 1971, 18, 115) und UDP-Glucase-Pyrophosphorylase aus Acanthamoeba castellanii (J. Biol. Chem., 1974, 249, 7832).

Bis auf die UDP-Glucose-Pyrophosphorylase aus Tetrahymena pyriformis (Arch. Biochern. Biophys., 1968, 127, 72) und aus Dictyostelium (J, Pauiovic et al., Dev. Genet. 9, 371-382 (1988); J.A. Ragheb et al., Nucleic Acid Res. 15, 3891-3896 (1987)) wurden erstaunlicherweise bisher keine Nukleotidyltransferasen in freilebenden, nicht-parasitären Protisten beschrieben.

Die vorliegende Erfindung betrifft somit ein nukleotidzuckersynthetisierendes Enzym, welches aus nicht-pathogenen Protisten erhältlich ist nämlich Tetrahymena, Colpidium, Potomacus oder Euglena, unter Ausnahme von UDP-Glucose-Pyrophosphorylase aus Tetrahymena pyriformis und aus Dictyostelium.

Zu den Vorteilen solcher nukleotidzuckersynthetisierender Enzyme aus der gemamten nichtpathogenen Protisten gehören:
1. Die Enzyme lassen sich leicht in großen Mengen über ferrnentative Verfahren herstellen.
2. Ein Stamm produziert häufig mehrere Nukleotidyitransferasen gleichzeitig.
3. Die Enzyme sind in der Regel stabil. Selbst nach mehrmaligem Einfrieren bleiben die Aktivitäten erhalten.
4. Die spezifischen Aktivitäten sind teilweise enorm hoch (siehe Tabelle 1).

Das erfindungsgemäße Enzym hat vorzugsweise die Aktivitäten einer UDP-N-Acetylgalactosamin-Pyrophosphorylase (UDP-GalNAc-Pyrophosphorylase), UDP-N-Acetylglucosamin-Pyrophosphorylase (EC Nr. 2.7.7.23), GDP-Fucose-Pyrophosphorylase (EC Nr. 2.7.7.30), GTP-Mannose-1-phasphat-guanyltransferase (EC Nr. 2.7.7.13), UDP-Glucose-Pyrophosphorylase (EC Nr. 2.7.7.9), einer UTP-Galactose-1-phosphat-uridyltransferase (EC Nr. 2.7.7.10), einer UTP-Xylose-1-phosphat-uridyltransferase (EC Nr. 2.7.7. 11) oder einer Nukleosidtriphosphat-hexose-1-phosphat-nukleotidyltransferase (EC Nr. 2.7.7.28). EC-Nummern gemäß Enzyme Nomenclature 1992, Academic Press, Inc., San Diego, New York, Boston.

Das erfindungsgemäße Enzym ist erhältlich aus Protisten der Klassen Ciliata (Gattungen Tetrahymena, Colpidium, Potomacus und Phytomastigophorea) oder aus den Mutanten dieser nichtparasitären Protisten.

Die Erfindung betrifft ferner ein Verfahren zur Herstellung eines solchen nukleotidzuckgrsynthetisierenden Enzyms.

Bei dem erfindungsgemäßen Verfahren werden die Protisten auf futterorganismenhaltigen, axenischen oder chemisch definierten Kulturmedien gezüchtet. Die Temperatur liegt dabei im Bereich von 15 - 45°C. Die Organismen können wahlweise in Form von Stand- bzw. Schüttelkulturen, in Spinnerflaschen oder Fermentern vermehrt werden, wobei die Fermentation vorzugsweise als batch-, fed-batch oder kontinuierliche Fermentation ausgelegt sein kann.

Vorzugsweise werden die Enzyme aus der Biomasse gewonnen. Die Biomasse kann z. B. über Tangentialfiltration, Sedimentation und Zentrifugation geerntet werden. Der anschließende Zellaufschluß kann z. B. wahlweise mittels Ultraschall- bzw. Ultraturraxapparaturen, Homogenisatoren oder durch Einfrieren durchgeführt werden.

Die enzymhaltigen Rohextrakte können direkt zur Herstellung von Nukleotidzuckern verwendet werden. Alternativ können die Enzyme zunächst über chromatographische Verfahren (z. B. Gelfiltration, lonenaustausch-, Affinitäts- und/oder Hydrophobe Interaktionschromatographie) und/oder Fällungen gereinigt werden. Demgemäß betrifft die vorliegende Erfindung auch die Verwendung des erfindungsgemäßen Enzyms zur Herstellung von Nukleotidzuckern.

Der Nachweis der nukleotidzuckersynthetisierenden Enzyme erfolgt über den sogenannten "nucleotidyltransferase substrate screening assay" (NUSSA) (Deutsche Patentanmeldung Nr. 195 17 093.8). Dieser Test gestattet die Identifizierung von nukleotidzuckersynthetisierenden Enzymen, indem er das bei Reaktion entstehende Produkt PPi mittels einer PPi-abhängigen Phosphofructokinase umsetzt. Die nachfolgende Enzymkaskade führt zur Entstehung von 2 Mol NAD pro Mol PPi. Je nachdem welches Nukleosidtriphosphat und welcher Zucker-1-Phosphat zu Beginn als Substrat eingesetzt wird, läßt sich zwischen den unterschiedlichen nukleotidzuckersynthetisierenden Enzymen differenzieren. Das Prinzip des NUSSA wird mittels folgenden Schemas erläutert:

**Nucleotidyltransferase Substrate Screening Assay (NUSSA)**

| | | |
|---|---|---|
| | 1 | |
| Zucker(-1-P) + NTP | 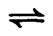 | N(M)DP-Zucker + PPi |
| | 2 | |
| PPi + Fru-6-P | 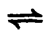 | Fr-1,6-P₂ + Pi |
| | 3 | |
| Fru-1,6-P₂ | 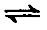 | DHAP + 3-P-Glycerinaldehyd |
| | 4 | |
| 3-P-Glycerinaldehyd | 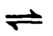 | DHAP |
| | 5 | |
| 2 DHAP + 2 NADH + 2 H⁽⁺⁾ | 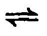 | 2 Glycerin-3-P + 2 NAD |

Enzyme
1 (nukleotidzuckersynthetisierendes Enzym (Pyrophosphorylase bzw. Nukleotidyltransferase) (EC 2.7.7.)
2 PPi-Phosphofructokinase (Kartoffel, EC 2.7.1.90)
3 Fructose-1,6-P₂Aldolase (Kaninchenmuskel, EC 4.1.2.13)
4 Triosephosphatisomerase (EC 5.3.1.1)
5 Glycerin-3-P-Dehydrogenase (EC 1.1.1.8)

Die Identifizierung der nukleotidzuckersynthetisierenden Enzyme kann neben dem NUSSA (Nucleotidyltransferase substrate screening assay) auch über HPLC-Verfahren und dünnschichtchromatographische Verfahren erfolgen.

### 1. HPLC

Die Proben werden gewonnen, wie z. B. in Beispiel 1 dargestellt. Anschließend wird eine geeignete Verdünnung der Probe in ein Puffersystem (z. B. 50 mM Tris/HCI) eingebracht, das die entsprechenden Nukleotidtriphosphate (z.B GTP, ATP) und die Zuolcerphosphate (z. B. Fucose-1-Phosphat) enthält. Die Inkubation kann e.B. 10 min bei 30° C betragen.
Die durch die Aktivität der in den Proben enthaltenden nukleotidzuckersynthesierenden Enzyme (z. B. GDP-Fucose Pyrophosphorylase) gebildeten Zuckernukleotide (z. B. GDP-Fucose) werden dann chromatographisch über HPLC (z.B. über eine RP₁₈-Säule) identifiziert, wobei die Gradientenelution z. B. über eine zunehmende Acetonitrilkonzentration durchgeführt wird. Lösung A besteht aus 5 mM Tetrabutylammaniumhydrogensulfat, 30 mM Kaliumdihydrogenphosphat und 4 % Acetonitril, pH 6. Lösung B besteht aus reinem Acetonitril.. Ein geeigneter Gradient ist z.B. 0-40% B, wobei die Flußraten vorzugsweise zwischen 1 und 2 ml/min liegen. Die Messung erfolgt mittels UV-Detector und Integrator. Alternativ zum Tetra-butylammoniumhydrogensulfat kann auch Tetrabutylammoniumbromid verwendet werden.

### 2. Dünnschichtchromatographie

Bei ausreichend hoher Konzentration der gebildeten Zuckernukleotide kann der Nachweis auch über DC erfolgen, Ein geeignetes Laufmittel ist z.B. Acetonitril/0,1 M NH₄Cl im Verhältnis 6:4. Als Sprühreagenz kann eine Lösung aus Naphthoresorcinol (20 mg), Diphenylamin (40 mg), Ethanol (10 ml) und Schwefelsäure (400 µl) verwendet werden. Als Platten werden TLC-glassplates, 0,25 mm Silicagel verwendet.

Die vorliegende Erfindung betrifft ferner auch die Verwendung der gemamten nichtparasitären Protisten, welche nukleotidsynthetisierende Enzyme produzieren, zur Herstellung von Nukleotidzuckern.

### Beispiele:

1. Tetrahymena thermophila: 2-1-Erlenmeyerkolben mit jeweils 500 ml Magermilchmedium (2% Magermilchpulver, 0,5% Hefeextrakt, 0,003% Sequestrene; nach Kiy & Tiedtke, 1992, Appl. Microbiol. Biotechnol., 37, 576) wurden 48 h bei 30° C und 100 rpm inkubiert. Die Kulturen wurden bei einer Zelldichte von 835.000 Zellen/ml über eine FILTRON-Tangentialfiltrationsanlage (Porengröße, 0,3 *µ*m) geerntet. Die aufkonzentrierte Zellsuspension wurde nochmals zentrifugiert (SORVALL RC-5B, GSA-Rotor, 10.000 rpm, 10 min). Das Zellpellet wurde durch Einfrieren bei -20° C aufgeschlossen.
Nach dem Auftauen wurde eine Probe in einer geeigneten Verdünnung in den NUSSA-Test eingebracht. Enzyme, die UDP-Glucose, UDP-N-Acetylglucosamin, UDP-Galactose, UDP-Galactosamin, UDP-N-Acetylgalactosamin, GDP-Fucose und GDP-Mannose synthetisieren, wurden in dem Rohextrakt identifiziert (Tab.1).
Überraschenderweise waren diese Enzyme z. T. (UDP-Glucose-, UDP-N-Acetylglucosamin-, UDP-Galactose-, UDP-N-Acetylgalactosamin- und GDP-Mannose-synthetisierende Enzyme) auch im zellfreien Kulturfiltrat vorhanden (Tab.2).
2. Tetrahymena thermophila: T. thermophila wird in einem 2-1-Rührfermenter (BRAUN BIOTECH INTERNATIONAL) bei 30° C kultiviert. Die Rührung erfolgte mittels eines Paddelimpellers, der pO₂ wurde bei 30% und der pH-Wert bei 7,0 gehalten. Als Medium wurde o. g. Magermilchmedium verwendet. Nach dem Erreichen der Stationärphase wurde wiederholt 5fach konz. Magermilchmedium zugeführt. Nach 10 Tagen wurden die Zellen bei einer Zelldichte von 7.000.000 Zellen/ml wie unter 1. beschrieben geerntet und weiter verarbeitet.
Mittels des NUSSA wurden die gleichen Enzyme wie unter 1. identifiziert (Tab. 3). Im zellfreien Kulturfiltrat wurden folgende Enzyme gefunden: (s. Tab. 4).
3. Tetrahymena setosa: Die Zellen wurden kultiviert wie unter 1. beschrieben. Bei einer Zelldichte von 1.320.000 Zellen/ml wurde die Kultur geerntet.
UDP-Glucose-, UDP-Glucosamin-, UDP-N-Acetylglucosamin-, UDP-Galactose-, UDP-Galactosamin-, UDP-N-Acetylgalactosamin-, GDP-Fucose- und GDP-Mannose-synthetisierende Enzyme wurden im Rohextrakt identifiziert (**Tab. 5**).
Im zellfreien Kulturfiltrat konnten UDP-Glucose-, UDP-Glucosamin-, UDP-N-Acetylglucosamin-, UDP-Galactose-, UDP-N-Acetylgalactosamin-, GDP-Fucose- und GDP-Mannose-synthetisierende Enzyme nachgewiesen werden (Tab. 6).
4. Tetrahymena pyriformis: T. pyriformis wurde bei 27°C und 60 rpm auf PPYS-Medium (1% Proteose-Pepton, 0,1% Hefeextrakt, 0,003% Sequestren) kultiviert. Bei einer Zelldichte von 500.000 Zellen/ml wurden die Kulturen wie unter 1. beschrieben geerntet und weiterverarbeitet.
Im Rohextrakt wurden UDP-Glucose-, UDP-N-Acetylglucosamin-, UDP-Galactose-, UDP-N-Acetylgalactosamin-, GDP-Fucose- und GDP-Mannose-synthetisierende Enzyme identifiziert (Tab. 7). Im zellfreien Kulturfiltrat wurden zusätzlich UDP-Glucosamin- und UDP-Galactosamin-synthetisierende Enzyme gefunden (Tab. 8).
5. Colpidium campylum: Dieses Ciliat wurde 3 d bei 25°C und 60 rpm auf Magermilchmedium inkubiert. Die Kultur wurde bei einer Zelldichte von 250.000 Zellen/ml wie unter 1. beschrieben geerntet und weiterverarbeitet.
UDP-Glucose-, UDP-Glucosamin-, UDP-N-Acetylglucosamin-, UDP-Galactose-, UDP-Galactosamin-, UDP-N-Acetylgalactosamin-, GDP-Fucose- und GDP-Mannose-synthetisierende Enzyme wurden im Rohextrakt identifiziert (Tab. 9).
Im zellfreien Kulturfiltrat wurden UDP-Glucose-, UDP-N-Acetylglucosamin-, UDP-Galactose- und GDP-Mannose-synthetisierende Enzyme detektiert (Tab. 10).
6. Potomacus pottsi: P. pottsi wurde 8 d als Standkultur bei 25°C auf Magermilchmedium inkubiert. Nachdem die Zellen eine Dichte von 160.000/ml erreicht hatten wurde die Kultur wie unter 1. beschrieben aufgearbeitet.
Der Rohextrakt enthielt UDP-Glucose-, UDP-Glucosamin-, UDP-N-Acetylglucosamin-, UDP-Galactose-, UDP-Galactosamin-, UDP-N-Acetylgalactosamin-, GDP-Fucose- und GDP-Mannose-synthetisierende Enzyme (Tab.11).
Im Kulturfiltrat konnten noch UDP-Glucose-, UDP-Glucosamin-, UDP-N-Acetylglucosamin-, UDP-Galactose-, UDP-Galactosamin- und UDP-N-Acetylgalactosamin-synthetisierende Enzyme detektiert werden (12).
7. Euglena gracilis: Dieser Flagellat wurde 7 d als Standkultur bei 25°C auf Medium 9 (1g/l Na-Acetat, 1g/l Fleischextrakt, 2g/l Bacto-Tryptone, 2g/l Hefeextrakt, 30ml/l Erdstammlösung) inkubiert. Bei einer Zelldichte 1.230.000 Zellen/ml wurden die Zellen wie unter 1. beschrieben geerntet. Der Zellausschluß erfolgte mit einem UltraTurrax.
UDP-Glucose-, UDF-Glucosamin-, UDP-N-Acetylglucosamin-, UDP-Galactose-, UDP-N-Acetylgalactosamin-, GDP-Fucose- und
GDP-Mannose-synthetisierende Enzyme wurden im Rohextrakt identifiziert (Tab. 13).
Im Kulturfiltrat wurden UDP-Glucose-, UDP-N-Acetylglucosamin-,
UDP-Galactose-, UDP-Galactosamin-, UDP-N-Acetylgalactosamin-,
GDP-Fucose- und GDP-Mannose-synthetisierende Enzyme gefunden (Tab. 14).

**Tabelle 1**

| | | | | | | |
|---|---|---|---|---|---|---|
| | | | NUSSA | | | |
| Enzym: | Rohextrakt, | Protein: | 22,3 mg/ml | | | |
| Quelle: | Tetrahymena thermophila | | | | | |
| Klasse: | | Ciliat | | | | |
| | | | | | | |
| Spezifische Aktivität (mU/mg) | | | | | | |
| | UTP | CTP | dTTP | ATP | GTP | |
| Glc-1-P | 440,00 | | | | | |
| GIcN-1-P | 0,00 | | | | | |
| GlcNAc-1-P | 130,00 | | | | | |
| GlcA-1-P | | | | | | |
| Gal-1-P | 240,00 | | | | | |
| GalN-1-P | 15,00 | | | | | |
| GalNAc-1-P | 47,00 | | | | | |
| GalA-1-P | | | | | | |
| Xyl-1-P | | | | | | |
| β-L-Fuc-1-P | | | | | 26,00 | |
| Man1-P | | | | | 38,00 | |

**Tabelle 2**

| | | | | | |
|---|---|---|---|---|---|
| | | | NUSSA | | |
| Enzym: | Filtrat, Protein: 0.065 mg/ml | | | | |
| Quelle: | Tetrahymena thermophila | | | | |
| Klasse: | | Ciliat | | | |
| | | | | | |
| Spezifische Aktivität (mU/mg) | | | | | |
| | UTP | CTP | dTTP | ATP | GTP |
| Glc-1-P | 92,00 | | | | |
| GlcN-1-P | 0,00 | | | | |
| GlcNAc-1-P | 62,00 | | | | |
| GlcA-1-P | | | | | |
| Gal-1-P | 46,00 | | | | |
| GalN-1-P | 0,00 | | | | |
| GalNAc-1-P | 1,50 | | | | |
| GalA-1-P | | | | | |
| Xyl-1-P | | | | | |
| β-L-Fuc-1-P | | | | | 00,00 |
| Man1-P | | | | | 21,50 |

**Tabelle 3**

| | | | | | |
|---|---|---|---|---|---|
| | | | NUSSA | | |
| Enzym: | Rohextrakt, Protein: 17,8 mg/ml | | | | |
| Quelle: | Tetrahymena thermophila | | | | |
| Klasse: | | Ciliat | | | |
| | | | | | |
| Spezifische Aktivität (mU/mg) | | | | | |
| | UTP | CTP | dTTP | ATP | GTP |
| Glc-1-P | 294,00 | | | | |
| GlcN-1-P | 0,00 | | | | |
| GlcNAc-1-P | 148,31 | | | | |
| GlcA-1-P | | | | | |
| Gal-1-P | 199,44 | | | | |
| GalN-1-P | 22,47 | | | | |
| GalNAc-1-P | 41,57 | | | | |
| GalA-1-P | | | | | |
| Xyl-1-P | | | | | |
| β-L-Fuc-1-P | | | | | 3,37 |
| Man1-P | | | | | 24,16 |

**Tabelle 4**

| | | | | | |
|---|---|---|---|---|---|
| | | | NUSSA | | |
| Enzym: | Filtrat, Protein: 0,42 mg/ml | | | | |
| Quelle: | Tetrahymena thermophila | | | | |
| Klasse: | | Ciliat | | | |
| | | | | | |
| Spezifische Aktivität (mU/mg) | | | | | |
| | UTP | CTP | dTTP | ATP | GTP |
| Glc-1-P | 21,43 | | | | |
| GicN-1-P | 6,19 | | | | |
| GlcNAc-1-P | 11,43 | | | | |
| GlcA-1-P | | | | | |
| Gal-1-P | 0,00 | | | | |
| GaIN-1-P | 0,00 | | | | |
| GaINAc-1-P | 0,00 | | | | |
| GaIA-1-P | | | | | |
| Xyl-1-P | | | | | |
| β-L-Fuc-1-P | | | | | 0,00 |
| Man1-P | | | | | 0,00 |

**Tabelle 5**

| | | | | | |
|---|---|---|---|---|---|
| | | | NUSSA | | |
| Enzym: | Rohextrakt, Protein: 14,3 mg/ml | | | | |
| Quelle: | Tetrahymena setosa | | | | |
| Klasse: | | Ciliat | | | |
| | | | | | |
| Spezifische Aktivität (mU/mg) | | | | | |
| | UTP | CTP | dTTP | ATP | GTP |
| Glc-1-P | 713,29 | | | | |
| GlcN-1-P | 36,36 | | | | |
| GlcNAc-1-P | 204,20 | | | | |
| GlcA-1-P | | | | | |
| Gal-1-P | 351,05 | | | | |
| GalN-1-P | 15,38 | | | | |
| GalNAc-1-P | 30,07 | | | | |
| GaIA-1-P | | | | | |
| Xyl-1-P | | | | | |
| β-L-Fuc-1-P | | | | | 16,08 |
| Man1-P | | | | | 39,16 |

**Tabelle 6**

| | | | | | |
|---|---|---|---|---|---|
| | | | NUSSA | | |
| Enzym: | Filtrat, Protein: 0,052 mg/ml | | | | |
| Quelle: | Tetrahymena setosa | | | | |
| Klasse: | | Ciliat | | | |
| | | | | | |
| Spezifische Aktivität (mU/mg) | | | | | |
| | UTP | CTP | dTTP | ATP | GTP |
| Glc-1-P | 48,08 | | | | |
| GlcN-1-P | 1,92 | | | | |
| GlcNAc-1-P | 15,38 | | | | |
| GlcA-1-P | | | | | |
| Gal-1-P | 23,08 | | | | |
| GalN-1-P | 0,00 | | | | |
| GalNAc-1-P | 26,92 | | | | |
| GalA-1-P | | | | | |
| Xyl-1-P | | | | | |
| β-L-Fuc-1-P | | | | | 36,54 |
| Man1-P | | | | | 19,23 |

**Tabelle 7**

| | | | | | |
|---|---|---|---|---|---|
| | | | NUSSA | | |
| Enzym: | Rohextrakt, Protein: 10.0 mg/ml | | | | |
| Quelle: | Tetrahymena pyriformis | | | | |
| Klasse: | | Ciliat | | | |
| | | | | | |
| Spezifische Aktivität (mU/mg) | | | | | |
| | UTP | CTP | dTTP | ATP | GTP |
| Glc-1-P | 509,00 | | | | |
| GlcN-1-P | 0,00 | | | | |
| GlcNAc-1-P | 96,00 | | | | |
| GlcA-1-P | | | | | |
| Gal-1-P | 312,00 | | | | |
| GalN-1-P | 0,00 | | | | |
| GalNAc-1-P | 2,00 | | | | |
| GalA-1-P | | | | | |
| Xyl-1-P | | | | | |
| β-L-Fuc-1-P | | | | | 34,00 |
| Man 1-P | | | | | 22,00 |

**Tabelle 8**

| | | | | | |
|---|---|---|---|---|---|
| | | | NUSSA | | |
| Enzym: | Filtrat, Protein: 0,054 mg/ml | | | | |
| Quelle: | Tetrahymena pyriformis | | | | |
| Klasse: | | Ciliat | | | |
| | | | | | |
| Spezifische Aktivität (mU/mg) | | | | | |
| | UTP | CTP | dTTP | ATP | GTP |
| Glc-1-P | 203,70 | | | | |
| GlcN-1-P | 22,22 | | | | |
| GlcNAc-1-P | 101,85 | | | | |
| GlcA-1-P | | | | | |
| Gal-1-P | 129,63 | | | | |
| GalN-1-P | 18,52 | | | | |
| GalNAc-1-P | 55,56 | | | | |
| GalA-1-P | | | | | |
| Xyl-1-P | | | | | |
| β-L-Fuc-1-P | | | | | 0,00 |
| Man1-P | | | | | 20,37 |

**Tabelle 9**

| | | | | | |
|---|---|---|---|---|---|
| | | | NUSSA | | |
| Enzym: | Rohextrakt, Protein: 8,8 mg/ml | | | | |
| Quelle: | Colpidium campylum | | | | |
| Klasse: | | Ciliat | | | |
| | | | | | |
| Spezifische Aktivität (mU/mg) | | | | | |
| | UTP | CTP | dTTP | ATP | GTP |
| Glc-1-P | 500,00 | | | | |
| GlcN-1-P | 9,09 | | | | |
| GlcNAc-1-P | 92,05 | | | | |
| GlcA-1-P | | | | | |
| Gal-1-P | 112,50 | | | | |
| GalN-1-P | 13,64 | | | | |
| GalNAc-1-P | 79,55 | | | | |
| GalA-1-P | | | | | |
| Xyl-1-P | | | | | |
| β-L-Fuc-1-P | | | | | 129,55 |
| Man1-P | | | | | 65,91 |

**Tabelle 10**

| | | | | | |
|---|---|---|---|---|---|
| | | | NUSSA | | |
| Enzym: | Filtrat, Protein: 0,096 mg/ml | | | | |
| Quelle: | Colpidium campylum | | | | |
| Klasse: | | Ciliat | | | |
| | | | | | |
| Spezifische Aktivität (mU/mg) | | | | | |
| | UTP | CTP | dTTP | ATP | GTP |
| Glc-1-P | 83,33 | | | | |
| GlcN-1-P | 0,00 | | | | |
| GlcNAc-1-P | 83,33 | | | | |
| GIcA-1-P | | | | | |
| Gal-1-P | 166,67 | | | | |
| GaIN-1-P | 0,00 | | | | |
| GaINAc-1-P | 0,00 | | | | |
| GalA-1-P | | | | | |
| Xyl-1-P | | | | | |
| β-L-Fuc-1-P | | | | | 0,00 |
| Man1-P | | | | | 437,50 |

**Tabelle 11**

| | | | | | |
|---|---|---|---|---|---|
| | | | NUSSA | | |
| Enzym: | Rohextrakt, Protein: 42,5 mg/ml | | | | |
| Quelle: | Potomacus pottsi | | | | |
| Klasse: | | Ciliat | | | |
| | | | | | |
| Spezifische Aktivität (mU/mg) | | | | | |
| | UTP | CTP | dTTP | ATP | GTP |
| Glc-1-P | 4,00 | | | | |
| GlcN-1-P | 0,50 | | | | |
| GlcNAc-1-P | 0,00 | | | | |
| GlcA-1-P | | | | | |
| Gal-1-P | 5,00 | | | | |
| GalN-1-P | 0,70 | | | | |
| GalNAc-1-P | 0,20 | | | | |
| GalA-1-P | | | | | |
| Xyl-1-P | | | | | |
| β-L-Fuc-1-P | | | | | 0,90 |
| Man1-P | | | | | 0,07 |

**Tabelle 12**

| | | | | | |
|---|---|---|---|---|---|
| | | | NUSSA | | |
| Enzym: | Filtrat, Protein: 0,11 mg/ml | | | | |
| Quelle: | Potomacus pottsi | | | | |
| Klasse: | | Ciliat | | | |
| | | | | | |
| Spezifische Aktivität (mU/mg) | | | | | |
| | UTP | CTP | dTTP | ATP | GTP |
| Glc-1-P | 45,00 | | | | |
| GlcN-1-P | 4,50 | | | | |
| GlcNAc-1-P | 5,50 | | | | |
| GlcA-1-P | | | | | |
| Gal-1-P | 8,20 | | | | |
| GaIN-1-P | 18,00 | | | | |
| GaINAc-1-P | 27,00 | | | | |
| GalA-1-P | | | | | |
| Xyl-1-P | | | | | |
| β-L-Fuc-1-P | | | | | 0,00 |
| Man1-P | | | | | 0,00 |

**Tabelle 13**

| | | | | | |
|---|---|---|---|---|---|
| | | | NUSSA | | |
| Enzym: | Rohextrakt, Protein: 12,8 mg/ml | | | | |
| Quelle: | Euglena gracilis | | | | |
| Klasse: Phytomastigophorea | | | | | |
| | | | | | |
| Spezifische Aktivität (mU/mg) | | | | | |
| | UTP | CTP | dTTP | ATP | GTP |
| Glc-1-P | 323,44 | | | | |
| GlcN-1-P | 11,72 | | | | |
| GlcNAc-1-P | 43,75 | | | | |
| GlcA-1-P | | | | | |
| Gal-1-P | 46,88 | | | | |
| GalN-1-P | 0,00 | | | | |
| GalNAc-1-P | 49,22 | | | | |
| GalA-1-P | | | | | |
| Xyl-1-P | | | | | |
| β-L-Fuc-1-P | | | | | 75,00 |
| Man1-P | | | | | 69,53 |

**Tabelle 14**

| | | | | | |
|---|---|---|---|---|---|
| | | | NUSSA | | |
| Enzym: | Filtrat, Protein: 0,059 mg/ml | | | | |
| Quelle: | Euglena gracilis | | | | |
| Klasse: | | Phytomastigophorea | | | |
| | | | | | |
| Spezifische Aktivität (mU/mg) | | | | | |
| | UTP | CTP | dTTP | ATP | GTP |
| Glc-1-P | 16,95 | | | | |
| GlcN-1-P | 0,00 | | | | |
| GlcNAc-1-P | 11,86 | | | | |
| GlcA-1-P | | | | | |
| Gal-1-P | 16,95 | | | | |
| GalN-1-P | 13,56 | | | | |
| GalNAc-1-P | 8,47 | | | | |
| GalA-1-P | | | | | |
| Xyl-1-P | | | | | |
| β-L-Fuc-1-P | | | | | 25,42 |
| Man1-P | | | | | 3,39 |

**Tabelle 15 Spezifische Aktivitäten ≥ 50 mU/mg im NUSSA.**

| | | | | | | | | |
|---|---|---|---|---|---|---|---|---|
| Probe | UDP-Gic | UDP-GlcN | UDP-GlcNAc | UDP-Gal | UDP-GalN | UDP-GaINAc | GDP-Fuc | GDP-Man |
| Tetrahymena thermophila | + | | + | + | | + | + | |
| Euglena gracilis | + | | + | + | | + | + | + |
| Tetrahymena pyriformis | + | | + | + | + | + | | + |
| Colpidium campylum | + | | + | + | | + | + | + |
| Tetrahymena setosa | + | | + | + | | | | |

1U ist die Enzymaktivität, welche zu Produktion von 1 *µ*mol Fructose-1,6-diphosphat pro Minute führt.

## Patentansprüche

1. Nukleotidzuckersynthetisierendes Enzym das eine GDP-Fucosepyrophosphorylase-Aktivität aufweist, erhältlich aus einem nichtparasitären Protisten ausgewählt aus den Klassen *Ciliata* oder *Phytomastigophorea,* **dadurch gekennzeichnet, dass** der nichtparasitären Protisten ausgewählt ist aus der Gruppe bestehend aus Tetrahymena, *Colpidium, Potomacus* oder *Euglena.*

2. Nukleotidzuckersynthetisierendes Enzym nach Anspruch 1, **dadurch gekennzeichnet, dass** der nichtparasitäre Protist eine Mutante ist.

3. Verfahren zur Herstellung eines nukleotidzuckersynthetisierenden Enzyms gemäß einem der Ansprüche 1 oder 2, **dadurch gekennzeichnet, dass** man eine Kultur des nichtparasitären Protisten auf einem Futterorganismus-haltigen, axenischen oder chemisch definierten Medium fermentiert und anschließend das Enzym isoliert.

4. Verfahren nach Anspruch 3, **dadurch gekennzeichnet, dass** die Kultur in einer Stand- oder Schüttelkultur fermentiert wird.

5. Verfahren nach Anspruch 3, **dadurch gekennzeichnet, dass** die Kultur in einer Spinnerflasche fermentiert wird.

6. Verfahren nach Anspruch 3, **dadurch gekennzeichnet, dass** die Kultur in einem Fermenter fermentiert wird.

7. Verfahren nach einem der Ansprüche 3 bis 6, **dadurch gekennzeichnet, dass** die Fermentation als batch-Fermentation ausgelegt ist.

8. Verfahren nach einem der Ansprüche 3 bis 6, **dadurch gekennzeichnet, dass** die Fermentation als fed-batch-Fermentation ausgelegt ist.

9. Verfahren nach einem der Ansprüche 3 bis 6, **dadurch gekennzeichnet, dass** die Fermentation als kontinuierliche Fermentation ausgelegt ist.

10. Verfahren nach einem der Ansprüche 3 bis 9, **dadurch gekennzeichnet, dass** das Enzym durch Tangentialfiltration, Sedimentation oder Zentrifugation der im wesentlichen aus Zellen der Protisten bestehenden Biomasse, anschließendem Zellaufschluss derselben und nachfolgender chromatographischer Reinigung des so gewonnenen Enzymrohextraktes isoliert wird.

11. Verwendung eines nukleotidzuckersynthetisierenden Enzyms nach einem der Ansprüche 1 oder 2 zur Herstellung eines Nukleotidzuckers.

12. Verwendung nach Anspruch 11, **dadurch gekennzeichnet, dass** als Enzym ein Zellhomogenisat eingesetzt wird.

13. Verwendung nach Anspruch 11, **dadurch gekennzeichnet, dass** als Enzym ein das gereinigte Enzym eingesetzt wird.

14. Verwendung eines Protisten gemäß einem der Ansprüche 1 oder 2, welcher ein oder mehrere nukleotidzuckersynthetisierende(s) Enzym(e) mit GDP-Fucosepyrophosphorylase-Aktivität gemäß einem oder mehreren der Ansprüche 1 oder 2 produziert, zur Herstellung eines Nukleotidzuckers.

## Claims

1. Nucleotide-sugar-synthesising enzyme having GDP fucose pyrophosphorylase activity, obtainable from a non-parasitic protist selected from the classes *Ciliata* and *Phytomastigophorea,* **characterised in that** the non-parasitic protist is selected from the group consisting of Tetrahymena, *Colpidium, Potomacus* and *Euglena.*

2. Nucleotide-sugar-synthesising enzyme according to claim 1, **characterised in that** the non-parasitic protist is a mutant.

3. Method of preparing a nucleotide-sugar-synthesising enzyme according to either one of claims 1 and 2, **characterised in that** a culture of the non-parasitic protist is fermented on an axenic or chemically defined medium containing feed organisms and the enzyme is then isolated.

4. Method according to claim 3, **characterised in that** the culture is fermented in a static or shaken culture.

5. Method according to claim 3, **characterised in that** the culture is fermented in a spinner flask.

6. Method according to claim 3, **characterised in that** the culture is fermented in a fermenter.

7. Method according to any one of claims 3 to 6, **characterised in that** the fermentation is configured as batch fermentation.

8. Method according to any one of claims 3 to 6, **characterised in that** the fermentation is configured as fed-batch fermentation.

9. Method according to any one of claims 3 to 6, **characterised in that** the fermentation is configured as continuous fermentation.

10. Method according to any one of claims 3 to 9, **characterised in that** the enzyme is isolated by tangential filtration, sedimentation or centrifugation of the biomass which consists substantially of cells of the protists, subsequent cell disruption thereof and subsequent chromatographic purification of the crude enzyme extract so obtained.

11. Use of a nucleotide-sugar-synthesising enzyme according to either one of claims 1 and 2 in the preparation of a nucleotide sugar.

12. Use according to claim 11, **characterised in that** a cell homogenate is used as enzyme.

13. Use according to claim 11, **characterised in that** the purified enzyme is used as enzyme.

14. Use of a protist according to either one of claims 1 and 2 which produces one or more nucleotide-sugar-synthesising enzyme(s) having GDP fucose pyrophosphorylase activity according to one or more of claims 1 and 2, in the preparation of a nucleotide sugar.

## Revendications

1. Enzyme synthétisant le sucre lié aux nucléotides, présentant une activité GDP-fucosepyrophosphorylase obtenue d'un protiste non parasitaire choisi des classes Ciliata ou Phytomastigophorea, **caractérisé en ce que**, le protiste non parasitaire est choisi d'un groupement consistant en Tétrahymena, Colpidium, Protomacus ou Englena.

2. Enzyme synthétisant le sucre lié aux nucléotides selon la revendication 1, **caractérisé en ce que**, le protiste non parasitaire est un mutant.

3. Procédé pour produire un enzyme synthétisant le sucre lié aux nucléotides selon l'une des revendications 1 ou 2, **caractérisé en ce qu'**une culture de protistes est fermentée sur un milieu axénique ou chimique avec une teneur riche en organismes fourragers, et en continuation l'enzyme est isolé.

4. Procédé selon la revendication 3, **caractérisé en ce que**, la culture est fermentée dans une culture stable ou agitée.

5. Procédé selon la revendication 3, **caractérisé en ce que**, la culture est fermentée dans un flacon de filage.

6. Procédé selon la revendication 3, **caractérisé en ce que**, la culture est fermentée dans une cuve de fermentation.

7. Procédé selon l'une des revendications 3 à 6, **caractérisé en ce que**, la fermentation est réalisée comme une fermentation en tas.

8. Procédé selon l'une des revendications 3 à 6, **caractérisé en ce que**, la fermentation est réalisée comme une fermentation en tas alimenté.

9. Procédé selon l'une des revendications 3 à 6, **caractérisé en ce que**, la fermentation est réalisée comme une fermentation continue.

10. Procédé selon l'une des revendications 3 à 9, **caractérisé en ce que**, l'enzyme est isolé par filtration tangentielle, la sédimentation ou la centrifugation de la biomasse consistant en essence de cellules de protistes suivie par la décomposition des cellules et en continuation la purification chromatographique de l'extrait d'enzyme brut ainsi extrait.

11. Utilisation d'une enzyme synthétisant le sucre lié aux nucléotides selon l'une des revendications 1 ou 2 pour produire un sucre lié aux nucléotides.

12. Utilisation selon la revendication 11, **caractérisée en ce qu'**on utilise comme enzyme une substance homogénéisatrice de cellules.

13. Utilisation selon la revendication 11, **caractérisée en ce qu'**on utilise comme enzyme un enzyme purifié.

14. Utilisation d'un protiste selon l'une des revendications 1 ou 2 qui produit l'un ou plusieurs enzyme(s) synthétisant le sucre lié aux nucléotides avec activité GDP - fucosepyrophosphorylase selon l'une ou plusieurs des revendications 1 ou 2 pour produire un sucre lié aux nucléotides.
